# EUROPEAN PATENT APPLICATION

(11) **EP 2 682 922 A1**
(43) Date of publication of application: **08.01.2014**
(21) Application number: 13173679.5
(22) Date of filing: 25.06.2013
(51) Int. Cl.: G08B 13/18, G01J 1/04, B65D 83/26, A61L 9/00, G08B 13/189

(54) **Omnidirectional presence detector**

(30) Priority: 05.07.2012 ES 201230737 U
(71) Applicant: Zyxtudio diseño e innovación SL, 46003 Valencia (ES)
(72) Inventor: Blasco Feo, Vicente, 46003 Valencia (ES)
(74) Representative: Soler Lerma, Santiago

(57) **Abstract**

Omnidirectional ambient light sensor for activating e.g. air freshener. Comprises transparent laminar element for receiving incident ambient light through its thin side, light sensors housed in holes on the laminar body, opaque elements housed in (separate) holes on the laminar body, electronic means for detecting a change in ambient light. Omnidirectionality is achieved by refraction and, optionally, reflection properties of the laminar body.

## Description

### SUBJECT MATTER OF THE INVENTION

Just as the name indicates, the subject of the present invention is a presence detector having a capacity to detect a presence in a 360° radius, thanks to the use of a lens that comprises a transparent laminated element, such as methacrylate, having a sufficient thickness, within which a series of sensors is installed in such a way that due to the refraction effect of the light waves entering through the lens, normally from the side, they propagate inside of the detector until activating the sensors.

The sector of the art to which the present invention pertains is that of presence detectors.

### BACKGROUND OF THE INVENTION

Some of the prior art that are going to be cited refer to detectors and their use in air freshening devices, as that is the preferred sector in which the applicant is going to apply them, notwithstanding other uses that they may be given.

With regard to presence detectors, there are different types in existence, infra-red, temperature or ambient light sensors, among others, could be mentioned.

Thus the subject of the present document refers to an ambient light sensor which detects changes in the light and interprets the existence of movement in the surroundings.

However, when these sensors are used in portable devices, as is the case of an air freshener, some problems may arise during use.

A common problem is that the sensor is left oriented towards a zone where the light does not change, or a dark area, for example facing a wall, which thus renders it ineffective.

Another similar problem occurs when the sensor is permanently exposed to intense light, for instance, if it is placed facing a lamp the sensor may lose its sensitivity.

If the light sensor loses its effectiveness, either due to a defect or excessive light, the device which it activates will not function correctly.

### DESCRIPTION

To overcome the problems explained above, the present subject invention refers to a very simple 360° sensor apt for adaptation to any device.

The 360° sensor comprises:
- A laminar element made of transparent material.
- One or more light sensitive heads lodged inside said laminar element.
- Electronic control elements

The sensor may also be equipped with:
- Light reflecting elements, preferably on their larger faces.
- Opaque elements that protect the sensitive heads.

The laminar elements essentially coincide with the cross-section, at least peripherally, of the device on which the sensor is installed.

The light enters through any of the exposed sides of the transparent laminar element and is propagated throughout its interior.

The fast propagation is due mainly to the transparent condition of the laminar element and the refraction of the light waves which, when traversing it, impact on the internal face of the larger surfaces of the laminar element.

This propagation will be enhanced if light reflecting elements are incorporated, such as a polishing of the larger faces of the laminar surface or a mirror, although this is optional because the refraction of the light when passing through one means to another is sufficient for correcting the functioning of the sensor.

The light propagated through the interior of the laminar surface impacts against the light sensitive heads that will detect changes in the intensity of the light.

The result is that the light reaches the sensitive head regardless of the point in which it enters the sensor..

To improve the performance of the sensor, sensitive heads can be added, regarding which a high efficiency performance has been verified using 3 sensitive heads arranged in a 120° formation.

The use of screens that limit the passage of light in order to protect each of the sensitive heads improves the performance of each one, given that the sensitive heads take real readings of the changes of light in their area of influence, and, in this way, through the combination of the data obtained from one sensitive head and another, the presence of movement can be detected in the space surrounding the sensor.

These screens do not necessarily have to be completely opaque, rather they can have different degrees of opacity.

When movement is detected the device that the sensor is connected to, which may be an air-freshener, alarm or any other type, is activated.

### BRIEF DESCRIPTION OF THE DRAWINGS

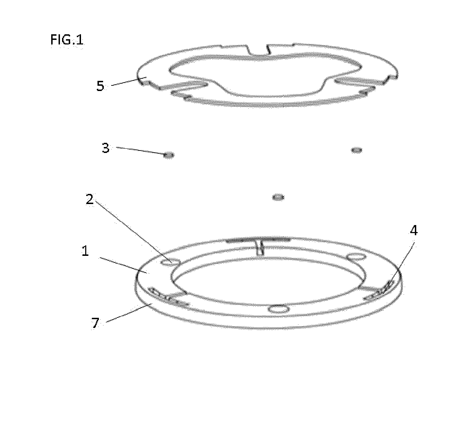

Figure 1 is an exploded view showing the main elements of the sensor in which laminar element made of transparent material (1) is visible, through edge (7) of which light enters, said element having several degrees of holes (2) apt for lodging in its interior sensitive heads (3), as well as several holes (4), in which the screens are lodged (not shown), and plate (5) that supports the electronic control elements.

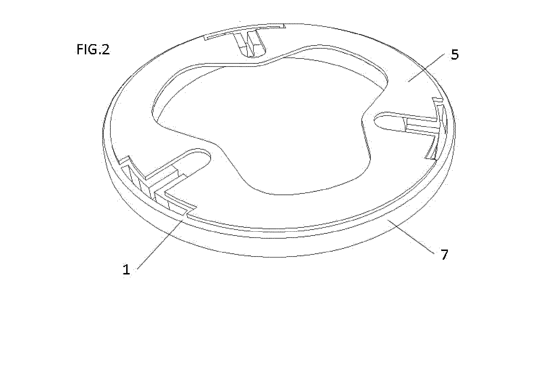

Figure 2 shows the assembly comprising laminar element made of transparent material (1), of which basically edge (7) is visible, and plate (5) that supports the electronic control elements.

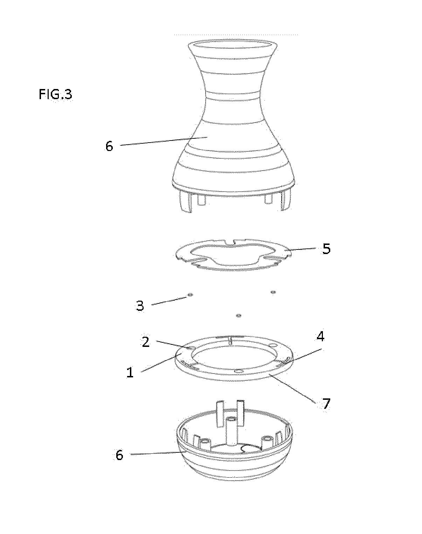

Figure 3 is an exploded view showing the sensor installed in air freshener device (6), specifically the device that is the subject of utility model U 201101001, and displaying laminar element made of transparent material (1), its edge (7), the holes (2) apt for housing in their interior sensitive heads (3), as well as another set of holes (4) in which the screens are lodged (not shown), and plate (5) that supports the electronic control elements.

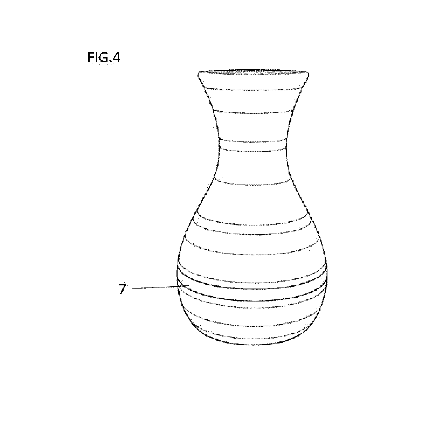

Figure 4 shows the the air freshener device displayed in an exploded view in Figure 3, but now in its configuration for use, with the sensor being perfectly integrated into the body of the same, showing only its edge (7)

### DESCRIPTION OF AN EMBODIMENT OF THE INVENTION

A non-excluding embodiment of the invention is proposed,merely for explanatory purposes.

The sensor comprises:
- A laminar element made of transparent material (7), such as methacrylate.
- Three holes (2) made on the inside of said laminar element, and arranged in 120° angles with respect of each other.
- In each of said holes (2) a sensitive head (3) is housed.
- Three hollow areas having a T shape (4) made in the transparent laminar element that houses in its interior the screens in a position apt for making a shadow fall on said sensitive head.
- An electronic control system supported by plate (5).

The sensor may be placed in any device meeting the requirement that for it to function sensor edges (7) must be exposed to the zone of influence that it is intended to cover.

The light impacts the sides of the sensor and is quickly propagated through its interior until reaching the sensitive heads.

The sensitive heads detect the variation in the light and electronically assess whether this change in the light has fallen only on one head or on more than one.

The sensor may or may not activate, depending on the variations of light detected, thanks to the electronic control systems on which it has been installed.

## Claims

1. PERIMETER PRESENCE DETECTOR, of the type that uses heads sensitive to changes of light, **characterised in that** it comprises:
• A laminar body of transparent material (1).
• At least one hole (2) in each laminar body.
• As many sensitive heads (3) as opacities, each sensitive head being housed in an opacity.
• Electronic control elements.
• Connecting means between the sensitive heads and the electronic control elements.

2. PERIMETER PRESENCE DETECTOR in accordance with claim 1, **characterised in that** the laminar body comprises at least one screen and, preferably, as many screens as sensitive heads.

3. PERIMETER PRESENCE DETECTOR; in accordance with claim 1, **characterised in that** at least one of its larger faces has light reflecting elements.

4. PERIMETER PRESENCE DETECTOR; in accordance with claim 1, characterised it comprises a support plate (5) for the electronic control elements.
